# FASCICULE DE BREVET EUROPEEN

(11) **EP 1 196 415 B1**
(45) Date de publication et mention de la délivrance du brevet: **08.01.2003**
(21) Numéro de dépôt: 00949572.2
(22) Date de dépôt: 29.06.2000
(51) Int. Cl.: C07D 457/04

(54) **PROCEDE DE PREPARATION DE L'ACIDE LYSERGIQUE**
VERFAHREN ZUR HERSTELLUNG VON LYSERGSÄURE
METHOD FOR PREPARING LYSERGIC ACID

(30) Priorité: 02.07.1999 FR 9908538
(43) Date de publication de la demande: 17.04.2002
(73) Titulaire: Aventis Pharma S.A., 92160 Antony (FR)
(72) Inventeur: GAULLIER, Jean-Claude, F-93460 Gournay Sur Marne (FR)
(74) Mandataire: Rousseau, Pierrick Edouard
(86) Numéro de dépôt international: FR0001820
(87) Numéro de publication internationale: WO01002395

(56) Documents cités:
- WOLFGANG OPPOLZER ET AL.: "Total synthesis of ( )-lysergic acid by an intramolecular imino-Diels-Alder reaction." HELVETICA CHIMICA ACTA., vol. 64, no. 47, - 1981 pages 478-481, XP002133277 VERLAG HELVETICA CHIMICA ACTA. BASEL., CH ISSN: 0018-019X cité dans la demande
- H. KOBEL ET AL.: "6-Methyl- -ergolen-8-carbonsaüre, ein neues Ergolinderivat aus Kulturen eines Stammes von Claviceps paspali Stevens et Hall." HELVETICA CHIMICA ACTA., vol. 47, no. 4, - 1964 pages 1052-1064, XP002133278 VERLAG HELVETICA CHIMICA ACTA. BASEL., CH ISSN: 0018-019X cité dans la demande

## Description

La présente invention concerne un nouveau procédé de préparation de l'acide lysergique par isomérisation de l'acide paspalique.

L'acide lysergique est un intermédiaire de synthèse de la nicergoline commercialisée pour améliorer des symptômes du déficit intellectuel pathologique du sujet âgé, dans les troubles rétiniens d'origine vasculaire, dans le traitement des accidents vasculaires cérébraux aigus.

La synthèse de la nicergoline nécessite d'utiliser un acide lysergique pur c'est-à-dire ne contenant qu'un faible pourcentage d'acide isolysergique.

Il est connu de préparer l'acide lysergique par isomérisation de l'acide paspalique au moyen de potasse (Helvetica Chimica Acta, 64, 47, 478 (1981) ou de soude (Helvetica Chimica Acta, 47, 115, 1052 (1964) et JP70013302) cependant ces procédés ne permettent pas d'obtenir industriellement soit de bons rendements soit un produit contenant de faibles quantités d'acide isolysergique.

Il a maintenant été trouvé et c'est ce qui fait l'objet de la présente demande un procédé permettant d'obtenir l'acide lysergique suffisamment pur et avec de très bons rendements par isomérisation d'acide paspalique.

Cette isomérisation s'effectue au moyen d'un hydroxyde de tétraalkyl(1-6C)ammonium. Il est également possible d'utiliser l'hydroxyde de tétraalkyl(1-6C)ammonium en mélange avec une petite quantité d'un hydroxyde de métal alcalin.

Comme hydroxyde de tétraalkyl(1-6C)ammonium on peut utiliser notamment l'hydroxyde de tétraméthylammonium, l'hydroxyde de tétraéthylammonium, l'hydroxyde de tétrabutylammonium.

De préférence, on utilise l'hydroxyde de tétrabutylammonium.

Comme hydroxyde de métal alcalin on peut utiliser notamment la soude et la potasse.

De préférence, on utilise la soude.

La mise en oeuvre du procédé est généralement réalisée au sein d'un solvant inerte tel que l'eau ou un alcool aliphatique (1-4C) (méthanol et éthanol par exemple) ou un mélange de ces solvants, à une température comprise entre 20°C et 60°C et, de préférence entre 25 et 35°C. Il est avantageux de laisser le milieu réactionnel à cette température pendant 20 à 30 heures et, en particulier, pendant 24 heures.

La quantité d'hydroxyde de tétraalkyl(1-6C)ammonium est généralement de 1,5 à 10 moles et, de préférence, de 2,5 moles par mole d'acide paspalique.

Lorsque l'on utilise un mélange d'hydroxyde de tétraalkyl(1-6C)ammonium et d'hydroxyde de métal alcalin, la quantité d'hydroxyde de tétraalkyl(1-6C)ammonium est généralement de 0,5 à 2 moles et, de préférence, de 1,5 mole et la quantité d'hydroxyde de métal alcalin est généralement de 4,5 moles à 0,5 mole et, de préférence de 1 mole par mole d'acide paspalique.

Encore plus préférentiellement, on opère en milieu aqueux, à une température de 28 à 32°C, soit en présence de 2,5 moles d'hydroxyde de tétrabutylammonium pour une mole d'acide paspalique soit en présence de 1,5 mole d'hydroxyde de tétrabutylammonium et de 1 mole de soude pour une mole d'acide paspalique.

L'acide lysergique est ensuite précipité par acidification du milieu réactionnel au moyen d'un acide minéral et, de préférence, au moyen d'acide sulfurique, et filtré. Il est avantageux d'acidifier à un pH d'environ 3-4 et, en particulier à 3,5 sans dépasser 30°C.

Les exemples suivants illustrent l'invention.

### EXEMPLE 1

A 780,1 g d'une solution d'hydroxyde de tétrabutylmammonium à 40% dans l'eau, sous courant d'azote, et sous agitation, on ajoute, rapidement 130 g d'acide paspalique. On porte à 30°C±2°C et laisse réagir à cette température pendant 20 heures. On refroidit le milieu réactionnel à environ 20°C et maintient à cette température pendant 3h30mn. On ajoute 918 g d'eau puis acidifie au moyen d'acide sulfurique à 95% jusqu'à pH3,5 en maintenant la température à environ 30°C. La masse réactionnelle est alors refroidie à 10°C±2°C et maintenue à cette température pendant 30 minutes. La masse est filtrée sur verre fritté sous un vide de 0,4 bar, lavée 3 fois par 300 ml d'eau puis séchée 14 heures à 75°C±2°C sous 20 mbar. On obtient ainsi 107 g d'acide lysergique contenant moins de 3% d'acide isolysergique, le RRi est égal à 80% (RRi = poids d'acide lysergique 100% isolé /poids paspalique 100% engagé).

### EXEMPLE 2

A 472,3 g d'une solution d'hydroxyde de tétrabutylmammonium à 40% dans l'eau, sous courant d'azote, et sous agitation, on ajoute, rapidement 130 g d'acide paspalique puis 316g d'une solution aqueuse de soude à 1,5N. On porte à 30°C±2°C et laisse réagir à cette température pendant 20 heures. On refroidit le milieu réactionnel à environ 20°C et maintient à cette température pendant 3h30mn. On ajoute 918 g d'eau puis acidifie au moyen d'acide sulfurique à 95% jusqu'à pH3,5 en maintenant la température à environ 30°C. La masse réactionnelle est alors refroidie à 10°C±2°C et maintenue à cette température pendant 30 minutes. La masse est filtrée sur verre fritté sous un vide de 0,4 bar, lavée 3 fois par 300 ml d'eau puis séchée 14 heures à 75°C±2°C sous 20 mbar. On obtient ainsi 107.8 g d'acide lysergique ne contenant que 2,8% d'acide isolysergique, le RRi est égal à 81,6%.

### Essais comparatifs :

### A - Préparation de l'acide lysergique avec de la soude seule.

5 g d'acide paspalique dans 100 ml d'une solution aqueuse de soude 2N sont chauffés à reflux pendant 2 heures. Après refroidissement, le pH du milieu réactionnel est amené à 5,5 par addition d'une solution aqueuse d'acide chlorhydrique et d'acide acétique glacial (20 ml d'eau, 10 ml d'acide chlorhydrique et 10 ml d'acide acétique). Le précipité est filtré, lavé par 3 fois 20 ml d'un mélange eau/méthanol (50:50) puis séché sous vide à 75°C. On obtient ainsi 3,15 g d'acide lysergique contenant 6,8% d'acide isolysergique, le RRi est égal à 59,3%.

### B - Préparation de l'acide lysergique avec de la potasse seule.

5 g d'acide paspalique dans 360g d'une solution de potasse 0,5N dans un mélange eau/éthanol (50:50) sont chauffés à reflux 1 heure. Après refroidissement, le pH du milieu réactionnel est amené à 5,5 par addition d'acide chlorhydrique 1N. Le précipité est filtré, lavé par 3. fois 20 ml d'un mélange eau/méthanol (50:50) puis séché sous vide à 75°C. On obtient ainsi 2,86 g d'acide lysergique contenant 1% d'acide isolysergique, le RRi est égal à 49,8%.

## Revendications

1. Procédé de préparation d'acide lysergique par isomérisation de l'acide paspalique **caractérisé en ce que** l'isomérisation est effectuée au moyen d'un hydroxyde de tétraalkyl( 1-6C)ammonium.

2. Procédé selon la revendication 1 dans lequel l'hydroxyde de tétraalkyl(1-6C)ammonium est choisi parmi l'hydroxyde de tétraméthylammonium, l'hydroxyde de tétraéthylammonium, l'hydroxyde de tétrabutylammonium.

3. Procédé selon l'une des revendications 1 ou 2 **caractérisé en ce que** l'on opère au sein d'un solvant inerte.

4. Procédé selon la revendication 3 **caractérisé en ce que** le solvant inerte est l'eau ou un alcool aliphatique (1-4C) ou un mélange de ces solvants.

5. Procédé selon l'une des revendications 1 à 4 **caractérisé en ce que** l'on utilise 1,5 à 10 moles d'hydroxyde de tétraalkyl(1-6C)ammonium pour 1 mole d'acide paspalique.

6. Procédé selon la revendication 5 **caractérisé en ce que** l'on utilise 2,5 moles d'hydroxyde de tétraalkyl(1-6C)ammonium pour une mole d'acide paspalique.

7. Procédé selon l'une des revendications 1 à 6 **caractérisé en ce que** l'on opère à une température comprise entre 20 et 60°C .

8. Procédé selon la revendication 7 **caractérisé en ce que** l'on opère à une température comprise entre 25 et 35°C.

9. Procédé selon la revendication 1 **caractérisé en ce que** l'on opère au moyen de 2,5 moles d'hydroxyde de tétrabutylammonium pour une mole d'acide paspalique, en milieu aqueux, à une température de 28 à 32°C.

10. Procédé selon l'une des revendications 1 à 4, 7 ou 8 **caractérisé en ce que** l'on opère en présence d'un hydroxyde de métal alcalin.

11. Procédé selon la revendication 10 **caractérisé en ce que** l'hydroxyde de métal alcalin est choisi parmi la soude et la potasse.

12. Procédé selon l'une des revendications 10 ou 11 **caractérisé en ce que** l'on utilise de 0,5 à 2 moles d'hydroxyde de tétraalkyl(1-6C)ammonium et de 4,5 à 0,5 moles d'hydroxyde de métal alcalin pour une mole d'acide paspalique.

13. Procédé selon l'une des revendication 10 à 12 **caractérisé en ce que** l'on utilise 1,5 mole d'hydroxyde de tétraalkyl(1-6C)ammonium et 1 mole d'hydroxyde de métal alcalin pour une mole d'acide paspalique.

14. Procédé selon la revendication 1 **caractérisé en ce que** l'on opère au moyen de 1,5 mole d'hydroxyde de tétrabutylammonium et 1 mole de soude pour une mole d'acide paspalique, en milieu aqueux, à une température de 28 à 32°C.

## Patentansprüche

1. Verfahren zur Herstellung von Lysergsäure durch Isomerisierung von Paspalsäure, **dadurch gekennzeichnet, daß** die Isomerisierung mit Hilfe eines Hydroxides von Tetraalkyl(1-6C)-ammonium durchgeführt wird.

2. Verfahren nach Anspruch 1, bei dem das Hydroxid von Tetraalkyl(1-6C)-ammonium unter dem Hydroxid von Tetramethylammonium, dem Hydroxid von Tetraethylammonium und dem Hydroxid von Tetrabutylammonium ausgewählt wird.

3. Verfahren nach einem der Ansprüche 1 oder 2, **dadurch gekennzeichnet, daß** man in einem inerten Lösungsmittel arbeitet.

4. Verfahren nach Anspruch 3, **dadurch gekennzeichnet, daß** das inerte Lösungsmittel Wasser oder ein aliphatischer (1-4C) Alkohol oder eine Mischung dieser Lösungsmittel ist.

5. Verfahren nach einem der Ansprüche 1 bis 4, **dadurch gekennzeichnet, daß** man 1,5 bis 10 mol Hydroxid von Tetraalkyl(1-6C)-ammonium pro 1 mol Paspalsäure verwendet.

6. Verfahren nach Anspruch 5, **dadurch gekennzeichnet, daß** man 2,5 mol Hydroxid von Tetraalkyl(1-6C)-ammonium pro 1 mol Paspalsäure verwendet.

7. Verfahren nach einem der Ansprüche 1 bis 6, **dadurch gekennzeichnet, daß** man bei einer Temperatur zwischen 20 °C und 60 °C arbeitet.

8. Verfahren nach Anspruch 7, **dadurch gekennzeichnet, daß** man bei einer Temperatur zwischen 25 °C und 35 °C arbeitet.

9. Verfahren nach Anspruch 1, **dadurch gekennzeichnet, daß** man mit Hilfe von 2,5 mol Hydroxid von Tetrabutylammonium pro 1 mol Paspalsäure, im wäßrigen Medium und bei einer Temperatur von 28 °C bis 32 °C arbeitet.

10. Verfahren nach einem der Ansprüche 1 bis 4, 7 oder 8, **dadurch gekennzeichnet, daß** man in Anwesenheit eines Alkalimetallhydroxides arbeitet.

11. Verfahren nach Anspruch 10, **dadurch gekennzeichnet, daß** das Alkalimetallhydroxid unter Natriumhydroxid und Kaliumhydroxid ausgewählt wird.

12. Verfahren nach einem der Ansprüche 10 oder 11, **dadurch gekennzeichnet, daß** man 0,5 bis 2 mol Hydroxid von Tetraalkyl-(1-6C)-ammonium und 4,5 bis 0,5 mol Alkalimetallhydroxid pro 1 Mol Paspalsäure verwendet.

13. Verfahren nach einem der Ansprüche 10 bis 12, **dadurch gekennzeichnet, daß** man 1,5 mol Hydroxid von Tetraalkyl-(1-6C)-ammonium und 1 mol Alkalimetallhydroxid pro 1 Mol Paspalsäure verwendet.

14. Verfahren nach Anspruch 1, **dadurch gekennzeichnet, daß** man mit Hilfe von 1,5 mol Hydroxid von Tetrabutylammonium und 1 mol Natriumhydroxid pro 1 mol Paspalsäure, im wäßrigen Medium und bei einer Temperatur von 28 °C bis 32 °C arbeitet.

## Claims

1. Process for preparing lysergic acid by isomerizing paspalic acid, **characterized in that** the isomerization is brought about using a tetra(C₁-C₆)-alkylammonium hydroxide.

2. Process according to Claim 1, in which the tetra(C₁-C₆)alkylammonium hydroxide is chosen from tetramethylammonium hydroxide, tetraethylammonium hydroxide and tetrabutylammonium hydroxide.

3. Process according to Claim 1 or 2 **characterized in that** it is carried out in an inert solvent.

4. Process according to Claim 3, **characterized in that** the inert solvent is water or an aliphatic (C₁-C₄)alcohol or a mixture of these solvents.

5. Process according to one of Claims 1 to 4, **characterized in that** from 1.5 to 10 mol of tetra(C₁-C₆)alkylammonium hydroxide are used for 1 mole of paspalic acid.

6. Process according to Claim 5, **characterized in that** 2.5 mol of tetra(C₁-C₆)-alkylammonium hydroxide are used for one mole of paspalic acid.

7. Process according to one of Claims 1 to 6, **characterized in that** it is carried out at a temperature between 20 and 60°C.

8. Process according to Claim 7, **characterized in that** it is carried out at a temperature between 25 and 35°C.

9. Process according to Claim 1, **characterized in that** it is carried out using 2.5 mol of tetrabutylammonium hydroxide for one mole of paspalic acid in an aqueous medium at a temperature of from 28 to 32°C.

10. Process according to one of Claims 1 to 4, 7 or 8, **characterized in that** it is carried out in the presence of an alkali metal hydroxide.

11. Process according to Claim 10, **characterized in that** the alkali metal hydroxide is chosen from sodium hydroxide and potassium hydroxide.

12. Process according to Claim 10 or 11, **characterized in that** from 0.5 to 2 mol of tetra(C1-C6)-alkylammonium hydroxide and from 4.5 to 0.5 mol of alkali metal hydroxide are used for one mole of paspalic acid.

13. Process according to one of Claims 10 to 12, **characterized in that** 1.5 mol of tetra(C₁-C₆)-alkylammonium hydroxide and 1 mol of alkali metal hydroxide are used for one mole of paspalic acid.

14. Process according to Claim 1, **characterized in that** it is carried out using 1.5 mol of tetrabutylammonium hydroxide and 1 mol of sodium hydroxide for one mole of paspalic acid in an aqueous medium at a temperature of from 28 to 32°C.
